(19) Europäisches Patentamt
European Patent Office
Office européen des brevets

(11) Veröffentlichungsnummer: **0 086 944**
**B1**

(12) # EUROPÄISCHE PATENTSCHRIFT

(45) Veröffentlichungstag der Patentschrift: **10.04.85**

(21) Anmeldenummer: **83100327.2**

(22) Anmeldetag: **15.01.83**

(51) Int. Cl.⁴: **C 07 C 43/15,** C 07 C 41/01, A 61 K 7/46, C 11 B 9/00, A 23 L 1/226

(54) **Neue ungesättigte Aether (I), Verfahren zu deren Herstellung, Verwendung von (I) als Riech- und/oder Geschmackstoffe und Riech- und/oder Geschmackstoffkompositionen mit einem Gehalt an (I).**

(30) Priorität: **27.01.82 CH 492/82**

(43) Veröffentlichungstag der Anmeldung: **31.08.83 Patentblatt 83/35**

(45) Bekanntmachung des Hinweises auf die Patenterteilung: **10.04.85 Patentblatt 85/15**

(84) Benannte Vertragsstaaten: **CH DE FR GB LI NL**

(56) Entgegenhaltungen: **EP - A - 0 045 453**

(73) Patentinhaber: **L. GIVAUDAN & CIE Société Anonyme, CH-1214 Vernier-Genève (CH)**

(72) Erfinder: **Kaiser, Roman, Weidstrasse 6, CH-8610 Uster (CH)**
Erfinder: **Lamparsky, Dietmar, Dr., Sonnhalde 8, CH-8602 Wangen (CH)**

(74) Vertreter: **Urech, Peter, Dr. et al, Grenzacherstrasse 124 Postfach 3255, CH-4002 Basel (CH)**

EP 0 086 944 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Riech- und/oder Geschmackstoffe. Es handelt sich dabei um die Verbindungen der Formel

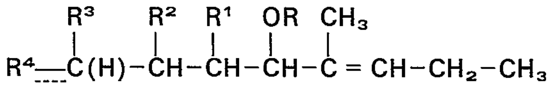

(I)

worin R $C_{1-4}$-Alkyl, 1-$C_{1-2}$-Alkoxy-$C_{1-4}$-alkyl oder $C_{2-4}$-Alkenyl bedeutet, $R^1$, $R^2$ und $R^3$ Wasserstoff, Methyl oder Äthyl darstellen und $R^4$ Wasserstoff, $C_{1-5}$-Alkyl oder $C_{2-4}$-Alkenyl oder $C_{1-4}$-Alkyliden darstellt, wobei 2 oder 3 der Reste $R^1$, $R^2$, $R^3$ und $R^4$ Wasserstoff sind.

R ist vorzugsweise $C_{1-4}$-Alkyl, insbesondere Methyl. Im Falle von R = Alkoxyalkyl ist der bevorzugte Rest 1'-Äthoxyäthyl. $R^1$ und $R^2$ sind vorzugsweise Wasserstoff. $R^3$ ist vorzugsweise Wasserstoff oder Methyl. $R^4$ ist vorzugsweise $C_{1-5}$-Alkyl.

Die Formel soll demgemäss $C_{1-4}$-Alkyläther, die ggf. in der 1'-Stellung eine zusätzliche $C_{1-2}$-Alkoxygruppe aufweisen, von sekundären, insbesondere von $C_{10-14}$-Alkoholen, denen das Strukturmerkmal $-CHOH-C(CH_3)=CH-CH_2-CH_3$ gemeinsam ist, umfassen.

Beispiele von $C_{1-15}$-Alkylresten sind Methyl, Äthyl, Propyl, Isopropyl, Butyl, Isobutyl, Amyl, Isoamyl.

Bevorzugt sind Methyl und Äthyl.

Beispiele von $C_{1-2}$-Alkoxyresten in der 1'-Stellung des Substituenten R sind Methoxy und Äthoxy.

Beispiele für $C_{2-4}$-Alkenylreste sind Vinyl, Propylen, Butylen, Allyl, Methallyl.

Bevorzugt sind Vinyl und Allyl.

Beispiele von $C_{2-4}$-Alkenyl- bzw. $C_{1-4}$-Alkylidenresten im Substituenten $R_4$ sind Vinyl, Allyl, Methallyl, 3'-Butenyl, 2'-Methyl-1'-propenyl, Methyliden, Äthyliden, Propyliden, Isopropyliden, Butyliden.

Bevorzugt sind Vinyl und Methyliden.

Die Formel I soll alle möglichen Stereoisomeren (cis- bzw. trans-Konfiguration an den Doppelbindungen) umfassen.

Die Erfindung betrifft ferner ein Verfahren zur Herstellung der Verbindungen der Formel I.

Dieses Verfahren ist dadurch gekennzeichnet, dass man eine Verbindung der Formel

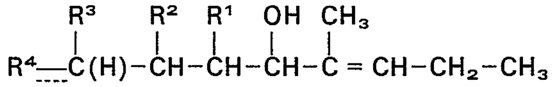

(II)

worin $R_1$, $R_2$, $R_3$ und $R_4$ obige Bedeutung besitzen, unter Einführung des Restes R auf an sich bekannte Weise veräthert.

Die Verätherung kann auf an sich bekannte Art und Weise bewerkstelligt werden. Zu diesem Zweck wird man also zweckmässigerweise die aus den entsprechenden Alkoholen der Formel II herstellbaren alkylieren, bzw. alkenylieren, s. z. B. „Organikum, Organisch-chemisches Grundpraktikum", 9. Aufl., VEB Deutscher Verlag der Wissenschaften, Berlin 1969, 222 ff.

Als Alkylierungsmittel eignen sich besonders Dialkylsulfate und Alkylhalogenide insbesondere Alkylbromid. Die Alkoholate stellt man zweckmässig durch Reaktion der zu Grunde liegenden Alkohole, mit einem Alkalimetall, wie z. B. Natrium oder Kalium, oder einem Alkalimetallhydrid, wie z. B. Natriumhydrid, in einem inerten Lösungsmittel wie Benzol, Toluol, Xylol usw. her.

Die Alkoxyalkylderivate der Formel I andererseits werden zweckmässigerweise durch Umsetzung der Alkohole II mit den Enoläthern der Formel

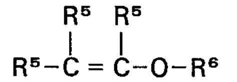

(III)

worin die Reste $R^5$ gleich oder verschieden sind und Wasserstoff oder Methyl oder Äthyl darstellen, mit der Bedingung, dass die Anzahl Kohlenstoffatome aller 3 Reste $R^5$ 2 nicht überschreitet, und $R^6$ für Methyl oder Äthyl steht, hergestellt.

Die Umsetzung wird zweckmässigerweise in Gegenwart von katalytischen Mengen von Mineralsäuren, wie z. B. Salzsäure, Schwefelsäure oder Phosphorsäure oder starker organischer Säuren, wie z. B. p-Toluolsulfonsäure(monohydrat) durchgeführt. Die Addition der Alkohole an die Doppelbindung der Enoläther bedarf im allgemeinen keines (zusätzlichen) Lösungsmittels, es kann jedoch ein Überschuss des Enoläthers diese Funktion übernehmen. Auch ist ein Zusatz aprotischer Lösungsmittel wie z. B. Hexan, Cyclohexan usw., ohne weiteres möglich. Die Reaktion verläuft normalerweise glatt bei Raumtemperatur, sie kann jedoch auch unter leichter Kühlung oder schwachem Erwärmen durchgeführt werden. Ein Temperaturbereich von 0 bis 80° C, bevorzugt +10 bis +40° C, trägt dieser Arbeitsweise Rechnung.

Die Ausgangsmaterialien der Formel II können dadurch erhalten werden, dass man 2-Methyl-2-pentenal mit einer Verbindung der Formel

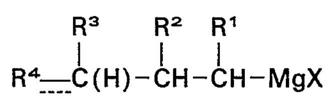

(IV)

worin $R^1$ bis $R^4$ obige Bedeutung besitzen und X für Halogen steht, umsetzt.

Als Halogenide IV kommen alle Halogenide in Frage, doch wird vorzugsweise das Bromid verwendet.

Die Umsetzung des Methylpentenals mit der Verbindung IV erfolgt zweckmässigerweise nach den an sich bekannten Methoden der Grignard-Reaktion, s. z. B. „Organikum, Org.-chem. Grundpraktikum", Nachdruck 15. Aufl., VEB Deutscher Verlag der Wissenschaften, Berlin 1977, 617 ff. Man arbeitet also zweckmässigerweise in Diäthyläther oder höheren Alkyläthern bzw. Tetrahydrofuran als Lösungsmittel und bei Temperaturen von ca. 0 bis 80° C.

Auf diese Weise fällt II in Form eines Isomerengemischs an, in welchem die trans-Form des Molekülteils $-CH(OH)-C(CH_3)=CH-CH_2-CH_3$ stark überwiegt.

Aus wirtschaftlichen Gründen wird man insbesondere dieses Isomerengemisch verwenden.

Die Verbindungen I weisen besondere organoleptische Eigenschaften auf, auf Grund derer sie sich vorzüglich als Riech- und/oder Geschmackstoffe eignen.

Die Erfindung betrifft demgemäss auch die Verwendung der Verbindungen I als Riech- und/oder Geschmackstoffe.

Das 5-Methoxy-4-methyl-3-decen beispielsweise besitzt einen sehr frischen, fruchtigen, an Äpfel, Blaubeeren und andere Beerenarten erinnernden, zugleich auch grün und krautig wirkenden Geruch. Die in der Kohlenstoffkette isomere Verbindung 5-Methoxy-2,6-dimethyl-6-nonen besitzt dagegen einen sehr süss-fruchtigen, pudrig wirkenden Geruch, während der entsprechende Äthyläther (5-Äthoxy-2,6-dimethyl-6-nonen) in erster Linie an Alkoholika (Whisky) erinnert und daneben aber noch zusätzlich blumig-würzige Aspekte aufweist. Das 5-[1'-Äthoxyäthoxy]-2,6-dimethyl-6-nonen zeigt grüne, blumige, leicht pudrige und sehr süss wirkende Geruchsnoten, die in der isomeren Verbindung 5-[1'-Äthoxyäthoxy]-4-methyl-3-decen durch bitterlich-grüne, an Citrus erinnernde, gleichzeitig aber auch schokoladenartige Geruchsnoten abgelöst werden.

Generell kann gesagt werden, dass die neuen Äther der Formel I interessante, hauptsächlich fruchtig-grüne Geruchsnoten aufweisen, deren blumige, würzige und pudrige Nuancen sie als ausgezeichnete, alkalistabile Riechstoffe sowie infolge der an verschiedene Fruchtarten, aber auch an Caramel und Schokolade erinnernden Geschmacksnoten auch als Geschmackstoffe, z. B. für (Nahrungsmittel-)Aromen erscheinen lassen.

Die Verbindungen der Formel I eignen sich aufgrund ihrer natürlichen Geruchsnoten insbesondere zur Modifizierung von z. B.

a) blumigen Kompositionen, in denen z. B. Kopfnoten belebt werden sollen (z. B. für Cologne-Typen u. ä., Extraits),

b) Kompositionen des Chypre- und Fougère-Typs, in denen frische, krautige, fruchtig-citrusartige Elemente betont werden sollen,

c) Tabak- und Holzkompositionen, in denen z. B. die Patchoulikomponente vorteilhaft eingekleidet wird und diese Modifikation auch in der Fondnote noch auftritt,

d) Kompositionen mit grünen Noten, wo insbesondere eine erwünschte Abrundung und harmonisierende Effekte erzielt werden.

Die Verbindungen I verbinden sich mit zahlreichen bekannten Riech- und/oder Geschmackstoffingredienzien natürlichen oder synthetischen Ursprungs, wobei die Palette der natürlichen Rohstoffe sowohl leicht- als auch mittel- und schwerflüchtige Komponenten, und diejenige der Synthetika Vertreter aus praktisch allen Stoffklassen umfassen kann, wie dies aus der folgenden Zusammenstellung ersichtlich ist:

– *Naturprodukte:* Basilikumöl, Baummoos-Absolue, Beifussöl, Bergamotteöl, Cassisknospen-Absolue, Castoreum, Cedernholzöl, Ciste Labdanum, Civette, Corianderöl, Eichenmoos, Elemiöl, Fichtennadelöl, Galbanum, Geraniumöl, Jasmin-Absolue und sein synthetischer Ersatz, Jonquille-Absolue, Labdanum, Lavendelöl, Mandarinenöl, Mastix-Absolue, Palmarosaöl, Patchouliöl, Petitgrainöl Paraguay, Sandelholzöl, Thymianöl, Weihrauch, Ylang-Ylang-Öl, Zitronenöl usw.

– *Alkohole:* Citronellol, Geraniol, cis-3-Hexenol, Linalool, Terpineol, Phenyläthylalkohol, Rhodinol, Sandela®(3-Isocamphyl-5-cyclohexanol), Dimetol®(2,6-Dimethylheptan-2-ol), Zimtalkohol synthetisch und sein Substitut usw.

– *Aldehyde:* α-Amylzimtaldehyd, Cyclamenaldehyd, Dodecanal, Heliotropin, α-Hexylzimtaldehyd, Hydroxycitronellal, 2,6,10-Trimethylundec-9-en-1-al (Adoxal®), Decanal, Undecanal, ω-Undecylenaldehyd usw.

– *Ketone:* Isoraldeine®(Isomethyl-α-jonon), α-Jonon, β-Jonon, 3-Prenylisocaranon, Vertofix® (=acetyliertes Cedernholzöl), p-Methylacetophenon usw.

– *Ester:* Amylsalicylat, Benzylacetat, Citronellylacetat, Bornylacetat, Terpenylacetat, Geranylacetat, cis-3-Hexenylacetat, cis-3-Hexenylbenzoat, 1-Methyl-2-sek.-butylcyclohexylacetat, Methyldihydrojasmonat, Phenoxyäthylisobutyrat, Phenyläthyltiglat, Cinnamylacetat, Styrallylacetat, 2,3,6,6-Tetramethylcyclohex-2-en-carbonsäureäthylester, 3,6,6-Trimethyl-2-äthylcyclohexy-2-en-carbonsäureäthylester, Vetivenylacetat, Cedrylacetat, p-tert.-Butylcyclohexylacetat usw.

– *Verschiedene:* Indol, Cumarin, Eugenol, Isobutylchinolin, Limonen, p-Menthan-8-thiol-3-on, 1-Methylcyclododecylmethyläther, γ-Nonalacton, δ-Decalacton, γ-Undecalacton, Ambrettemoschus, Galaxolid®(1,3,4,6,7,8-Hexahydro-4,6,6,7,8,8-hexamethylcyclopenta-γ-2-benzopyran), Ketonmoschus, Musk 174® (12-Oxahexadecanolid) usw.

Die Verbindungen der Formel I lassen sich in weiten Grenzen einsetzen, die beispielsweise von 0,1 (Detergentien) bis 50° C (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen, z. B. mit bis zu 60% neuartige Komplexe aufbauen kann. Die bevorzugten Konzentrationen bewegen sich zwischen 0,5 und 20%. Die mit I hergestellten Kompositionen lassen sich für alle Arten von parfümierten Verbrauchsgütern einsetzen (Eaux de Cologne, Eaux de Toilette, Extraits, Lotionen, Cremes, Shampoos, Seifen, Salben, Puder, Zahnpasten, Mundwässer, Desodorantien, Detergentien, Tabak usw.).

Die Verbindungen I können demgemäss bei der Herstellung von Kompositionen und – wie obige Zusammenstellung zeigt – unter Verwendung einer breiten Palette bekannter Riechstoffe, verwendet werden. Bei der Herstellung solcher Kompositionen können die oben aufgeführten bekannten Riechstoffe nach (dem Parfümeur bekannter) Art und Weise verwendet werden, wie z. B. aus W.A. Poucher, „Perfumes, Cosmetics and Soaps", *2*, 7. Aufl., Chapman und Hall, London, 1974, hervorgehend.

Als Geschmackstoffe können die Verbindungen I beispielsweise zur Erzeugung bzw. Verbesserung, Verstärkung, Steigerung oder Modifizierung von Frucht- (z. B. Melone, Quitte, Pfirsich, Aprikose) oder Beerenaromen, oder von Schokoladearomen in Nahrungsmitteln (Joghurt, Süsswaren usw.); in Genussmitteln (Tee, Tabak usw.) und Getränken (Alkoholika usw.) verwendet werden.

Die ausgeprägten geschmacklichen Qualitäten der Verbindungen I ermöglichen die Verwendung in geringen Konzentrationen. Eine geeignete Dosierung umfasst beispielsweise den Bereich von 0,01 bis 100 ppm, vorzugsweise von 0,1 bis 20 ppm im Fertigprodukt, d. h. dem aromatisierten Nahrungsmittel, Genussmittel oder Getränk.

Bei der Aromatisierung von beispielsweise Tabak kann die Dosierung jedoch auch höher liegen und einen grösseren Bereich umfassen, beispielsweise den Bereich von 1 bis 1000 ppm, vorzugsweise 50 bis 500 ppm.

Die Verbindungen können auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw. in essbaren Materialien verteilen lassen. Sie enthalten beispielsweise etwa 0,1 bis 10, insbesondere 0,5 bis 3 Gew.-%. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung bereits enthalten oder können leicht der Literatur entnommen werden, wie z. B. J. Merory, „Food Flavorings, Composition, Manufacture and Use", 2. Aufl., The Avi Publishing Company, Inc., Westport, Conn. (1968), oder G. Fenaroli, „Fenaroli's Handbook of Flavor Ingredients", 2. Aufl., vol. 2, CRC-Press, Inc., Cleveland, Ohio (1975).

Für die Herstellung solcher üblicher Gebrauchsformen kommen beispielsweise folgende Trägermaterialien, Verdickungsmittel, Geschmackstoffverbesserer, Gewürze und Hilfsingredientien usw. in Frage:

Gummi arabicum, Tragant, Salze, oder Brauereihefe, Alginate, Carrageen oder ähnliche Absorbentien; Indole, Maltol, Dienale, Gewürzoleoresine, Raucharomen; Gewürznelken, Diacetyl, Natriumcitrat; Mononatriumglutamat, Dinatriuminosin-5'-monophosphat (IMP), Dinatriumguanosin-5-phosphat (GMP); oder spezielle Aromastoffe, Wasser, Äthanol, Propylenglykol, Glycerin.

*Beispiel 1:*

Zu 1,54 g einer 55 bis 60%igen Natriumhydriddispersion in 25 ml Toluol werden bei 80° C unter Rühren 5,0 g 4-Methyl-3-decen-5-ol gelöst in 25 ml Toluol im Verlauf von 30 min zugetropft. Das Gemisch wird 12 h bei 80° C gerührt, auf 40° C abgekühlt und tropfenweise mit 5,6 g Dimethylsulfat versetzt, wobei die Temperatur wieder auf 80° C ansteigt. Zur Vervollständigung der Umsetzung wird noch 3 h bei 80° C gerührt. Nach Abkühlung auf 40° C werden 10 ml Äthanol zugegeben. Es wird kurz zum Sieden erhitzt und dann mit kalter 2N NaOH versetzt. Das Reaktionsprodukt wird in Hexan aufgenommen, neutral gewaschen, an 250 g Kieselgel zur Entfernung des Öls der eingesetzten NaH-Dispersion chromatographiert und das so erhaltene Rohprodukt fraktioniert destilliert. Es werden 4,1 g 5-Methoxy-4-methyl-3-decen (= 76% d.Th.) erhalten, Siedepunkt 100 bis 105°/14 Torr, $n_D^{20}$ = 1,4337.

Geruch: sehr frisch, fruchtig, grün, apfelartig, Blaubeeren, Brombeeren, krautig.

IR: 1670, 1094, 854 $cm^{-1}$

NMR: ~0,90 (2 überlagerte t, GH); 1,52 (s, 3H); 3,18 (s, 3H); 3,39 (t, 1H); 5,32 (t, LH)

MS: 184 ($M^+$, 1), 113(100), 81(39), 69(11), 55(17), 41(13)

Das Ausgangsmaterial kann wie folgt erhalten werden.

In einer für Grignard-Reaktionen üblichen Apparatur werden 2,4 g (= 0,1 G-Atom) Magnesium in 50 ml absolutem Äther vorgelegt. Unter Rühren und Schutzgasatmosphäre (Stickstoff) werden anschliessend 15,0 g (= 0,1 mol) n-Amylbromid in 50 ml absolutem Äther so zugetropft, dass der Äther nach Anspringen der Reaktion ständig schwach siedet. Nach Beendigung der Zugabe wird noch 30 min bei Rückflusstemperatur gehalten, dann auf 10° C abgekühlt und eine Lösung von 7,85 g (= 0,08 mol) 2-Methyl-2-pentenal in 20 ml absolutem Äther zugetropft. Zwecks Beendigung der Reaktion wird 12 h bei Raumtemperatur weitergerührt. Nach Zersetzung des Grignard-Komplexes mit gesättigter Ammoniumchloridlösung und Eis wird die überstehende ätherische Lösung mit gesättigter Kochsalzlösung gewaschen und anschliessend getrocknet. Nach Abdampfen des Lösungsmittels verbleiben 13,6 g Rohprodukt, die fraktioniert destilliert werden. Man erhält so 8,9 g reines 4-Methyl-3-decen-5-ol vom Siedepunkt 103° C/12 Torr, $n_D^{20}$ = 1,4499.

IR: 3340, 2958 + 2924, 2888 + 1858, 1670, 1460, 1024, 854 $cm^{-1}$

NMR: (360 MHz): 0,89 (t, 3H); 0,965 (t, 3H); 1,595 (s, 3H); 2,03 (m, 2H); 3,96 (t, 1H), 5,36 (t, 3H)

MS: m/e = 170 ($M^+$, 6), 155(1), 141(18), 128(2), 109(2), 99(100), 81(19), 71(15), 55(19), 43(42)

*Beispiel 2:*

In einem Reaktionsgefäss mit Rührer, Thermometer, Rückflusskühler und Tropftrichter werden 6,13 g einer 55 bis 60%igen Natriumhydriddispersion in 100 ml Benzol vorgelegt und unter Rühren einer Lösung von 20,0 g 2,6-Dimethyl-6-nonen-5-ol in 100 ml Benzol bei Raumtemperatur im Verlauf von 30 min zugetropft. Zur Vervollständigung des Umsatzes wird 12 h bei Rückflusstemperatur gehalten. Anschliessend wird auf 20° C abgekühlt. Bei dieser Temperatur werden 20,7 g Dimethylsulfat allmählich so zugetropft, dass die Temperatur 35° C nicht übersteigt (exotherme Reaktion). Nach beendeter Zugabe wird das dicker werdende Reaktionsgemisch noch 4 h auf Rückflusstemperatur erhitzt, dann abgekühlt und mit 25 ml Methanol versetzt. Nach 5 min wird eine Lösung von 6,7 g Natriumhydroxid in 40 ml Wasser zugetropft und erneut 2 h auf Rückflusstemperatur erhitzt. Nach Abkühlung wird das Reaktionsprodukt in Äther aufgenommen, mit Wasser neutral gewaschen und die Lösungsmittel über eine Vigreux-Kolonne abdestilliert. Das verbleibende Rohprodukt wird unter Zusatz von 0,1 g Natriumcarbonat am Wasserstrahlvakuum rektifiziert und liefert 13,3 g (entsprechend 61,7% d.Th.) reines 5-Methoxy-2,6-dimethyl-6-nonen vom Siedepunkt 78° C/12 Torr, $n_D^{20}$ = 1,4339.

Geruch: süss, fruchtig, pudrig, grün.

IR: 1670, 1384, 1368, 1094, 855 $cm^{-1}$

NMR: ~0,90 (überlagert d und t, zusammen 9H); 1,54 (s, 3H); 3,18 (s, 3H); 3,39 (t, 1H); 5,32 (t, 1H)

MS: 184 ($M^+$, 1), 113(100), 81(38), 55(17), 41(12)

Das Ausgangsmaterial kann wie folgt erhalten werden:

In 500 ml absolutem Äther werden 69,5 g (= 2,9 G-Atome) Magnesium vorgelegt. Anschliessend wird die Lösung von 438 g Isoamylbromid, das gemäss Gaschromatogramm ca. 1,5%N-Amylbromid enthält, in 1,2 l absolutem Äther so zugetropft, dass die exotherme Reaktion den Äther ständig beim Siedepunkt hält. Nach Beendigung der Zugabe wird noch 30 min bei Rückflusstemperatur gehalten. Die Grignard-Lösung wird alsdann auf 10° C abgekühlt. Dann werden 236,5 g (= 2,41 mol) 2-Methyl-2-pentenal in 600 ml absolutem Äther innert 40 min zugetropft, die Temperatur liegt so dauernd zwischen 10 und 20° C. Zwecks Beendigung der Reaktion wird noch 1 h bei Rückflusstemperatur gehalten. Das Reaktionsgemisch wird anschliessend auf Eis gegeben, mit wässeriger Salzsäure zersetzt, die ätherische Lösung mit Soda und gesättigter Kochsalzlösung neutral gewaschen und hierauf getrocknet. Das nach Abdestillieren des Lösungsmittels verbleibende Rohprodukt (480 g) wird über eine Widmer-Kolonne fraktioniert destilliert und ergibt 312 g 2,6-Dimethyl-6-nonen-5-ol (III) vom Siedepunkt 62° C/0,05 Torr, $n_D^{25}$ = 1,4479, das gemäss Gaschromatogramm (Carbowax, 130° C) 1,5% 4-Methyl-3-decen-5-ol enthält.

IR: 3350, 2956 + 2930, 2866, 1670, 1468, 1386, 1368, 1012, 856 $cm^{-1}$

NMR (60 MHz): 0,88 + 0,90 (zusammenfallend, 9H); 1,59 (s, 3H); 2,02 (t, 2H); 3,93 (t, 1H); 5,33 (t, 1H)

MS: m/e = 170(7), 141(16), 123(4), 99(100), 81(34), 71(19), 55(36), 43(91)

*Beispiel 3:*

Analog den Beispielen 1 und 2 werden aus 4,6- bzw. 4,7-Dimethyl-3-nonen-5-ol die isomeren Methyläther

a) 5-Methoxy-4,6-dimethyl-3-nonen (Siedepunkt 76° C/12 Torr, $n_D^{20}$ = 1,4355, Geruch: blumig, fruchtig, grün, krautig, erdig, sehr diffusiv), bzw.

b) 5-Methoxy-4,7-dimethyl-3-nonen (Siedepunkt 70° C/12 Torr, $n_D^{20}$ = 1,4354, Geruch: krautig, erdig, grün)

in vergleichbaren Ausbeuten erhalten.

Die Ausgangsmaterialien für a und b können wie folgt erhalten werden.

a) In einer für Grignard-Reaktionen üblichen Apparatur werden 28,3 g (1,18 G-Atom) Magnesium in 200 ml Äther vorgelegt. Unter Rühren und Schutzgasatmosphäre ($N_2$) werden anschliessend 178,1 g (1,18 mol) 2-Brompentan in 500 ml absolutem Äther so zugetropft, dass der Äther nach Anspringen der Reaktion ständig schwach siedet. Nach beendeter Zugabe wird noch 30 min bei Rückflusstemperatur gehalten, dann auf 10° C abgekühlt und eine Lösung von 96,1 g (0,98 mol) 2-Methyl-2-pentenal in 300 ml Äther während 30 min so zugetropft, dass die Reaktionstemperatur ständig zwischen 10 und 20° C liegt. Zwecks Beendigung der Reaktion wird noch 1 h rückflussiert, dann der Grignard-Komplex mit gesättigter Ammoniumchloridlösung und Eis zersetzt, die überstehende ätherische Lösung mit gesättigter Kochsalzlösung gewaschen und anschliessend getrocknet. Nach Abdampfen des Lösungsmittels verbleiben 176 g Rohprodukt, die fraktioniert destilliert werden. Man erhält so 119 g (71,4%) olfaktisch gutes 4,6-Dimethyl-3-nonen-5-ol vom Siedepunkt 92° C/12 Torr.

IR: 3380, 2958, 2924, 2865, 1670, 1460, 1378, 1300, 1005, 854

NMR: 0,70-1,20 (2t und 1d, gegenseitig überlagert, 9H); 1,58 (s, 3H); 2,02 (m, 2H); 3,67 (m, 1H); 5,34 (t, J ~6,5, 1H)

MS: 170 ($M^+$, 2), 141(2), 128(3), 123(1), 109(1), 99(100), 81(25), 71(12), 55(11), 43(72)

b) Es wird das Grignard-Reagens, welches durch Reaktion von 11,8 g (0,49 G-Atom) Magnesium in 50 ml Äther und 67,13 g (0,49 mol) Isobutylbromid in 250 ml Äther erhalten wurde, mit 40,0 g (0,41 mol) 2-Methyl-2-pentenal in 100 ml Äther umgesetzt. Die fraktionierte Destillation des Rohproduktes (94 g) über eine 20-cm-Widmerkolonne ergibt 44,4 g (69,4%) olfaktisch gutes 4,7-Dimethyl-3-octen-5-ol vom Siedepunkt 47 bis 48° C/0,04 Torr.

IR: 3350, 2958, 2930, 2865, 1670, 1468, 1383, 1367, 1305, 1050, 1000, 856

NMR: 0,80-1,20 (1d + 1t, 9H); 1,60 (s, 3H); 2,02 (m, 2H); 4,08 (t, J ~6,5, 1H); 5,37 (t, J ~6,5, 1H)

MS: 156 ($M^+$, 9), 127(24), 114(16), 109(6), 99(100)

*Beispiel 4:*

Analog den Beispielen 1 bis 3 wird aus 4,6-Dimethyl-3-octen-5-ol 5-Methoxy-4,6-dimethyl-3-octen erhalten, Siedepunkt 65° C/12 Torr, $n_D^{20}$ = 1,4329

Geruch: sehr grün, an Pflanzenstengel erinnernd.

Das Ausgangsmaterial kann wie folgt hergestellt werden:

Es wird das Grignard-Reagens, welches durch Reaktion von 18,7 g (0,77 G-Atom) Magnesium in 100 ml Äther und 105,4 g (0,77 mol) 2-Brombutan in 300 ml Äther erhalten wurde, mit 62,72 g (0,64 mol) 2-Methyl-2-pentenal in 200 ml Äther umgesetzt. Die fraktionierte Destillation des Rohproduktes (98,8 g) über eine 20-cm-Widmerkolonne ergibt 68,4 g (68,5%) olfaktisch gutes 4,6-Dimethyl-3-octen-5-ol vom Siedepunkt 83° C/12 Torr.

IR: 3400, 2960, 2930, 2870, 1670, 1460, 1378, 1300, 1035, 1000, 858

NMR: 0,70-1,10 (2t + 1d, gegenseitig überlagert, 9H); 1,58 (s, 3H); 2,02 (m, 2H); 3,68 (m, 1H); 5,37 (t, J ~6,5, 1H)

MS: 156 ($M^+$, 1), 109(1), 99(54), 81(20), 71(10), 57(12), 55(15), 43(100), 41(22), 39(7)

*Beispiel 5:*

Analog Beispiel 2 werden aus 20,0 g 2,6-Dimethyl-6-nonen-5-ol und 25,3 g Diäthylsulfat 10,9 g 5-Äthoxy-2,6-dimethyl-6-nonen erhalten, Siedepunkt 85° C/12 Torr, $n_D^{20}$ = 1,4315.

Geruch: nach Whisky, grün, fruchtig, blumig, würzig.

IR: 1670, 1386, 1368, 1116, 1086, 855 $cm^{-1}$

MS: 198 ($M^+$, 1), 127(100), 99(39), 81(27), 71(10), 55(17), 43(55)

*Beispiel 6:*

Analog den Beispielen 1 und 5 werden aus 20 g 4-Methyl-3-decen-5-ol und 27,1 g Diäthylsulfat 18,6 g 5-Äthoxy-4-methyl-3-decen vom Siedepunkt 37° C/0,03 Torr, $n_D^{20}$ = 1,4330, gewonnen.

Geruch: fettig, grün, beerenartig.

*Beispiel 7:*

Zu 6,2 g einer 55 bis 60%igen Natriumhydriddispersion in 100 ml Toluol werden bei 80° C im Verlauf von 30 min 20 g 4-Methyl-3-decen-5-ol, gelöst in 100 ml Toluol zugetropft. Das Reaktionsgemisch wird 12 h bei 80° C gerührt, dann auf 50° C abgekühlt und tropfenweise mit einer Lösung von 24,1 g n-Butylbromid in 50 ml Toluol versetzt. Anschliessend wird 6 h bei Rückflusstemperatur gehalten. Zur Aufarbeitung wird das Reaktionsgemisch bei Raumtemperatur mit Wasser versetzt, die organische Schicht abgetrennt und mit Wasser neutral gewaschen, getrocknet und nach Zusatz von 0,2 g Kaliumcarbonat fraktioniert destilliert. Man erhält 20,7 g 5-Butoxy-4-methyl-3-decen, Siedepunkt 60° C/0,03 Torr, $n_D^{20}$ = 1,4378.

Geruch: caramelartig, schwach, fettig-fruchtig.

IR: 1670, 1094 $cm^{-1}$

MS: 226 ($M^+$, 1), 155(66), 99(100), 81(18), 71(7), 55(13), 43(36)

Das Ausgangsmaterial kann wie folgt erhalten werden.

In einer für Grignard-Reaktionen üblichen Apparatur werden 2,4 g (= 0,1 G-Atom) Magnesium in 50 ml absolutem Äther vorgelegt. Unter Rühren und Schutzgasatmosphäre (Stickstoff) werden anschliessend 15,0 g (= 0,1 mol) n-Amylbromid in 50 ml absolutem Äther so zugetropft, dass der Äther nach Anspringen der Reaktion ständig schwach siedet. Nach Beendigung der Zugabe wird noch 30 min bei Rückflusstemperatur gehalten, dann auf 10° C abgekühlt und eine Lösung von 7,85 g (= 0,08 mol) 2-Methyl-2-pentenal in 20 ml absolutem Äther zugetropft. Zwecks Beendigung der Reaktion wird 12 h bei Raumtemperatur weitergerührt. Nach Zersetzung des Grignard-Komplexes mit gesättigter Ammoniumchloridlösung und Eis wird die überstehende ätherische Lösung mit gesättigter Kochsalzlösung gewaschen und anschliessend getrocknet. Nach Abdampfen des Lösungsmittels verbleiben 13,6 g Rohprodukt, die fraktioniert destilliert werden. Man erhält so 8,9 g reines 4-Methyl-3-decen-5-ol vom Siedepunkt 103° C/12 Torr, $n_D^{20}$ = 1,4499.

IR: 3340, 2958 + 2924, 2888 + 1858, 1670, 1460, 1024, 854 $cm^{-1}$

NMR (360 MHz): 0,89 (t, 3H); 0,965 (t, 3H); 1,595 (s, 3H); 2,03 (m, 2H); 3,96 (t, 1H); 5,36 (t, 3H)

MS: m/e = 170 ($M^+$, 6), 155(1), 141(18), 128(2), 109(2), 99(100), 81(19), 71(15), 55(19), 43(42)

*Beispiel 8:*

Analog Beispiel 7 werden 10 g 4-Methyl-3-decen-5-ol mit 12,1 g Allylbromid umgesetzt. Nach üblicher Aufarbeitung werden 6,9 g 5-Allyloxy-4-methyl-3-decen erhalten, Siedepunkt 51° C/0,03 Torr, $n_D^{20}$ = 1,4447.

Geruch: fettig, fruchtig, grün, Quitte.

IR: 1670, 1646, 1090, 998, 924, 860 $cm^{-1}$

MS: 210 ($M^+$, 1), 139(100), 97(77), 69(32), 41(74)

*Beispiel 9:*

In einem Reaktionsgefäss werden 10,0 g 2,6-Dimethyl-6-nonen-5-ol und 12,7 g Äthylvinyläther vorgelegt und unter Rühren vorsichtig mit 0,1 ml konzentrierter Salzsäure versetzt. Das Reaktionsgemisch erwärmt sich leicht; es wird 24 h bei Raumtemperatur gerührt, dann in Hexan aufgenommen und mit wässeriger Natriumhydrogencarbonatlösung und Wasser neutral gewaschen, getrocknet und im Wasserstrahlvakuum fraktioniert destilliert. Man erhält so 8,3 g (= 58,3% d.Th.) 5-(1'-Äthoxyäthoxy)-2,6-dimethyl-6-nonen vom Siedepunkt 110° C/12 Torr, $n_D^{20}$ = 1,4340.

Bei der gaschromatographischen Analyse erscheint entsprechend den beiden asymmetrischen Kohlenstoffatomen im Molekül ein Doppelpeak im Verhältnis 1:1.

Geruch: grün, blumig, süss, pudrig.

IR: 1670, 1124, 1096, 1058, 1012, 960 $cm^{-1}$

NMR (360 MHz): 0,87 (d, 6H); 0,96 (t, 3H); 1,20 (t, 3H); 1,26 (t, 3H); 1,59 (s, 3H); 2,04 (m, 2H); 2,85-3,70 (m, total 3H); 4,59 (m, 1H); 5,31 (m, 1H)

MS: 171, 97, 81, 73(100), 55, 45

*Beispiel 10:*

Analog Beispiel 9 werden 10,0 g 4-Methyl-3-decen-5-ol und 12,7 g Äthylvinyläther zu 9,7 g 5-(1'-Äthoxyäthoxy)-4-methyl-3-decen umgesetzt, Siedepunkt 115° C/12 Torr, $n_D^{20}$ = 1,4349.

Geruch: grün, bitterlich, citrusartig, an Schokolade erinnernd.

*Beispiel 11:*

10 g 2,6-Dimethyl-6-nonen-5-ol und 12,7 g Isopropenylmethyläther werden gemischt und mit 3 Tropfen konzentrierter Salzsäure versetzt. Das Gemisch wird anschliessend 15 h bei Rückflusstemperatur gehalten, abgekühlt, in Hexan aufgenommen und mit Natriumhydrogencarbonatlösung und Wasser neutral gewaschen. Man erhält nach Abdestillieren des Lösungsmittels 14,1 g rohes 5-(1'-Methoxy-1'-methyläthoxy)-2,6-dimethyl-6-nonen, welche im Vakuum der Wasserstrahlpumpe rektifiziert werden (Siedepunkt 109° C/12 Torr, $n_D^{20}$ = 1,4379).

Das gemischte Acetal zersetzt sich im Gaschromatographen und muss daher dünnschichtchromatographisch (Elutionsmittel: Gemisch Hexan/Pentan/Chloroform/Essigester im Verhältnis 25/25/50/5) auf Reinheit geprüft werden, $R_f$ = 0,60.

IR: 1664, 1384, 1374, 1218, 1082, 1024 $cm^{-1}$

NMR (360 MHz): δ = 0,85 (d, 3H); 0,87 (d, 3H); 0,94 (t, 3H); 1,30 (s, 3H); 1,33 (s, 3H); 1,50 (m, 4H); 1,56 (s, 3H); 2,02 (m, 2H); 3,18 (s, 3H); 3,97 (t, 1H); 5,28 (t, 1H)

MS: 212 ($M-CH_2O$), 153, 109, 97, 81, 73(100), 55, 43

*Beispiel 12:*

Analog Beispiel 11 erhält man aus 10 g 4-Methyl-3-decen-5-ol 8,8 g 5-(1'-Methoxy-1'-methyläthoxy)-4-methyl-3-decen vom Siedepunkt 111° C/12 Torr, $n_D^{20}$ = 1,4391

Geruch: fettig, citrusartig, seifig.

*Beispiel 13:*

Zu 5,90 g (~0,14 mol) einer 55 bis 60%igen Natriumhydriddispersion in 100 ml Toluol werden unter Rühren 20,0 g (0,11 mol) 4-Methyl-3-undecen-5-ol, gelöst in 35 ml Toluol im Verlaufe von 30 min zugetropft. Das Gemisch wird 12 h bei Rückflusstemperatur gerührt, dann auf 40° C abgekühlt, im Verlaufe von 20 min mit 18,4 g (0,13 mol) Methyljodid versetzt und zur Vervollständigung der Reaktion noch weitere 5 h bei 60° C gerührt. Das Reaktionsgemisch wird vorsichtig mit 5 ml Methanol und anschliessend mit 100 ml Wasser versetzt, die organische Phase mit 100 ml Äther verdünnt, mit gesättigter Kochsalzlösung gewaschen und mit Natriumsulfat getrocknet. Nach Abdampfen der Lösungsmittel verbleiben 24 g Rohprodukt, die fraktioniert destilliert werden. Man erhält so 13,5 g (62,5%) olfaktisch gutes 5-Methoxy-4-methyl-3-undecen vom Siedepunkt 97 bis 98° C/12 Torr, $n_D^{20}$ = 1,4366

Geruch: grün, blätterartig, fettig.

IR: 1670, 1125, 1102, 1092, 942, 850 $cm^{-1}$

MS: 198 ($M^+$, 3), 169(3), 113(100), 97(2), 81(9), 55(2), 41(3)

*Beispiel 14:*

Analog Beispiel 1 werden 20,0 g (0,11 mol) 4-Methyl-3-undecen-5-ol mit Natriumhydrid in Toluol ins Alkoholat übergeführt und anschliessend mit 14,2 g (0,13 mol) Äthylbromid zum Äthyläther umgesetzt. Nach analogem Aufarbeiten und Abdampfen der Lösungsmittel verbleiben 28,5 g Rohprodukt, die fraktioniert destilliert werden. Man erhält so 14,4 g (62,2%) olfaktisch gutes 5-Äthoxy-4-methyl-3-undecen vom Siedepunkt 104° C/12 Torr, $n_D^{20}$ = 1,4351.

Geruch: ätherisch, bitterlich, grün, leicht fruchtig.

IR: 1670, 1112, 1085, 1000, 850 $cm^{-1}$

MS: 212 ($M^+$, 2), 183(3), 127(100), 99(10), 81(9), 69(3), 55(9), 43(15), 43(16)

*Beispiel 15:*

Analog Beispiel 1 werden 10,0 g (0,047 mol) 4-Methyl-3-tridecen-5-ol mit Natriumhydrid in Toluol ins Alkoholat übergeführt und anschliessend mit 8,50 g (0,060 mol) Methyljodid zum Methyläther umgesetzt. Nach analogem Aufarbeiten und Abdampfen der Lösungsmittel verbleiben 13,6 g Rohprodukt, die fraktioniert destilliert werden. Man erhält so 5,5 g (51,8%) olfaktisch gutes 5-Methoxy-4-methyl-3-tridecen vom Siedepunkt 114° C/12 Torr, $n_D^{20}$ = 1,4407.

Geruch: nussig, fettig, grün, leicht holzig.

IR: 1670, 1130, 1095, 850 $cm^{-1}$

MS: 226 ($M^+$, 2), 197(3), 157(2), 113(100), 81(5), 69(2), 55(3), 41(3)

*Beispiel 16:*

Analog Beispiel 1 werden 11,0 g (0,065 mol) 2,6-Dimethyl-1,6-nonadien-5-ol mit Natriumhydrid in Toluol ins Alkoholat übergeführt und anschliessend mit Methyljodid zum Methyläther umgesetzt. Nach analogem Aufarbeiten und Abdampfen der Lösungsmittel verbleiben 26,9 g Rohprodukt, die fraktioniert destilliert werden. Man erhält so 6,2 g (52,3%) olfaktisch gutes 5-Methoxy-2,6-dimethyl-1,6-nonadien vom Siedepunkt 83 bis 84° C/12 Torr, $n_D^{20}$ = 1,4458.

Geruch: grün, beerig, fruchtig.

IR: 3062, 1650, 1155, 1099, 1065, 940, 881, 850 $cm^{-1}$

MS: 182 ($M^+$, 1), 150(15), 126(6), 113(100), 96(4), 81(19), 55(6), 41(6)

*Beispiel 17:*

Analog Beispiel 1 werden 11,0 g (0,06 mol) 4-Methyl-3,8-(Z)-undecadien-5-ol mit Natriumhydrid in Toluol ins Alkoholat übergeführt und anschliessend mit 10,7 g (0,075 mol) Methyljodid zum Methyläther umgesetzt. Nach analogem Aufarbeiten und Abdampfen der Lösungsmittel verbleiben 15,0 g Rohprodukt, die fraktioniert destilliert werden. Man erhält so 6,2 g (52,4%) olfaktisch gutes 5-Methoxy-4-methyl-3,8-(Z)-undecadien vom Siedepunkt 100°C/12 Torr, $n_D^{20}$ = 1,4472.

Geruch: grün, lederig, tranig.

IR: 1665, 1097, 1062, 940, 852, 720 $cm^{-1}$

MS: 196 ($M^+$, 1), 167(6), 135(2), 113(100), 95(2), 85(4), 81(10), 69(3), 55(3), 41(6)

*Beispiel 18:*

In einem Reaktionsgefäss werden 15,0 g (0,081 mol) 4-Methyl-3-undecen-5-ol und 17,6 g (0,244 mol) Äthylvinyläther vorgelegt und unter Rühren mit 0,1 ml konzentrierter Salzsäure versetzt. Das Reaktionsgemisch erwärmt sich leicht und wird 24 h bei Raumtemperatur gerührt, dann in Hexan aufgenommen, mit wässeriger Natriumhydrogencarbonatlösung und Wasser neutral gewaschen und mit Natriumsulfat getrocknet. Nach Abdampfen der Lösungsmittel verbleiben 22,0 g Rohprodukt, die fraktioniert destilliert werden. Man erhält so 13,7 g (65,6%) olfaktisch gutes 5-(1'-Äthoxyäthoxy)-4-methyl-3-undecen vom Siedepunkt 87 bis 89°C/0,06 Torr, $n_D^{20}$ = 1,4366. Das Produkt zeigt bei der gaschromatographischen Analyse entsprechend den beiden asymmetrischen Kohlenstoffatomen im Molekül einen Doppelpeak im Verhältnis von 1:1.

Geruch: fruchtig, grün, fettig.

IR: 1130, 1090, 1058, 1025, 1010, 850 $cm^{-1}$

MS: 256 ($M^+$, 1), 183(3), 171(19), 167(11), 111(2), 97(2), 83(3), 81(4), 73(100), 55(5), 45(18)

*Beispiel 19:*

A) Blumige Base

| | *Gewichtsteile* |
|---|---|
| Terpineol | 260 |
| Hydroxycitronellal | 220 |
| Zimtalkoholsubstitut | 120 |
| Phenyläthylalkohol | 100 |
| Cinnamylformiat | 20 |
| Linalool | 15 |
| Terpenylacetat | 10 |
| Ketonmoschus | 10 |
| Geranylacetat | 10 |
| Jasmin synth. | 10 |
| Eugenol | 5 |
| Indol 10% in Dipropylenglykol (DPG) | 5 |
| Decanol 10% in DPG | 5 |
| p-Methylacetophenon | 5 |
| Undecalacton | 5 |
| | 800 |

Durch den Zusatz von 200 Teilen 5-Methoxy-4-methyl-3-decen wird die ursprüngliche Base überraschenderweise viel fruchtiger, der Kopf der neuen Base wirkt grüner und frischer und besitzt eine weitaus kräftigere Ausstrahlung als der Kopf der Base ohne Zusatz.

B) Parfümerie-Base vom Fougère-Typ

| | *Gewichtsteile* |
|---|---|
| Lavendelöl | 200 |
| Amylsalicylat | 200 |
| Baum-Moos 50% in Propylenglykol | 80 |
| Citronellol | 80 |
| Geraniol | 80 |
| Ketonmoschus | 80 |
| Bergamotteöl | 80 |
| α-Jonon | 80 |
| α-Hexylzimtaldehyd | 30 |
| Eugenol | 20 |
| Metambrate® GIV (1-Acetoxy-1-methyl-2-sec.-butylcyclohexan) | 20 |
| | 950 |

Gibt man zu dieser Fougère-Base 50 Teile 5-Methoxy-4-methyl-3-decen, so wirkt die Base viel frischer und krautiger. Sie erhält eine weitaus typischere Lavendel-Fougère-Note.

C) Holzige Base

| | *Gewichtsteile* |
|---|---|
| Bergamotteöl | 200 |
| Patchouliöl | 200 |
| Sandelholzöl | 200 |
| Cedrylacetat | 100 |
| Methyldihydrojasmonat | 70 |
| Methyljonon | 50 |
| p-tert.-Butylcyclohexylacetat | 50 |
| Basilikumöl | 30 |
| | 900 |

Gibt man zu obiger Holz-Base 10 Teile 5-Methoxy-2,6-dimethyl-6-nonen, so resultiert eine Base mit völlig neuer Richtung. Die neue Base wirkt weniger krautig-würzig, sie zeigt sehr stark in Richtung Chypre, mit süsslicher Nuance, Charakter: Richtung Lebkuchen. Erstaunlicherweise ist dieser Effekt sogar noch nach 24 h festzustellen. In der Original-Base ist andererseits nach dieser Zeit die Patchouli-Note spitz hervorgetreten. Durch den Zusatz wirkt die Base dagegen weicher, die Patchouli-Note wird vorteilhaft eingekleidet.

D) Grün-blumige Parfümerie-Base

| | *Gewichtsteile* |
|---|---|
| Hydroxycitronellal | 130 |
| Bergamotteöl | 130 |
| α-Jonon | 100 |
| α-Hexylzimtaldehyd | 100 |
| Heliotropin | 70 |
| Styrallylacetat | 80 |
| Ylang synthetisch | 80 |
| Benzylacetat | 80 |
| Phenyläthylalkohol | 80 |
| 2,6-Dimethylheptan-2-ol | 60 |
| Jasmin synthetisch | 20 |
| Decalacton | 5 |
| | 935 |

Gibt man zu dieser Base 65 Teile 5-Methoxy-2,6-dimethyl-6-nonen, so wird im 24 h-Wert eine sehr angenehme blumig-kosmetische Note der Originalkomposition unterstrichen; diese Note erinnert an Goldlack.

E) Parfümerie-Base (grün-blumig)

| | Gewichtsteile |
|---|---|
| Hydroxycitronellal | 130 |
| Bergamotteöl | 130 |
| α-Jonon | 100 |
| α-Hexylzimtaldehyd | 100 |
| Heliotropin | 70 |
| Styrallylacetat | 80 |
| Ylang synthetisch | 80 |
| Benzylacetat | 80 |
| Phenyläthylalkohol | 80 |
| 2,6-Dimethylheptan-2-ol | 60 |
| Jasmin synthetisch | 20 |
| Decalacton | 5 |
| | 935 |

Durch Zugabe von 65 Teilen 5-(1'-Äthoxyäthoxy)-4-methyl-3-decen wird in dieser Base eine interessante grüne Kopfnote erzielt, die gleichzeitig sehr angenehm an natürlichen Veilchenduft erinnert und sich durch eine zuvor nicht beobachtete Frische auszeichnet.

F) Parfümerie-Base vom Chypre-Typ

| | Gewichtsteile |
|---|---|
| Methyl-1-methylcyclododecyläther | 200 |
| Bergamotteöl | 150 |
| Hydroxycitronellal | 100 |
| Fichtenöl Pumillon | 80 |
| Citronellol | 80 |
| Petitgrainöl | 60 |
| Musc 174® GIV (12-Oxahexadecanolid) | 60 |
| Corianderöl | 40 |
| Galbanumöl | 40 |
| Zedernholzöl | 40 |
| Patchouliöl | 40 |
| Zitronenöl | 40 |
| Elemiöl | 10 |
| Eichenmoos entfärbt | 20 |
| | 960 |

Gibt man zu dieser Chypre-Base 40 Teile 5-(1'-Äthoxyäthoxy)-4-methyl-3-decen, so erhält diese eine sehr angenehme frisch-fruchtige Note, welche an Citrus erinnert. Die Base hat nun ausserdem viel mehr Volumen, d. h. die Strahlungskraft der neuen Komposition ist erheblich grösser.

G) Base vom Typ Cologne

| | Gewichtsteile |
|---|---|
| Myrascone® GIV (2-Äthyl-3,6,6-trimethyl-2-cyclohexen-1-carbonsäureäthylester) | 80 |
| Bergamotteöl | 80 |
| Galaxolide® GIV (1,3,4,6,7,8-Hexahydro-4,6,6,7,8,8-hexamethyl-cyclopenta-γ-2-benzopyran) | 60 |
| Hydroxycitronellal | 60 |
| Madrox® GIV (1-Methyl-1-methoxy-cyclododecan) | 60 |
| Bornylacetat | 40 |
| Sandalore® GIV [3-Methyl-5-(2,2,3-trimethylcyclopent-3-en-1-yl)-pentan-2-ol] | 30 |
| Ketonmoschus | 40 |
| Givescone® GIV (2-Äthyl-6,6-dimethyl-2-cyclohexan-1-carbonsäureäthylester) | 20 |
| Petitgrainöl | 20 |
| Corps Cassis® GIV (p-Menthan-8-thiol-3-on) | 5 |
| Baum-Moos absolut | 5 |
| Dipropylenglykol | 450 |
| | 950 |

Die Zugabe von 50 Teilen 5-Äthoxy-2,6-dimethyl-6-nonen bewirkt eine sehr schöne Verstärkung der Kopfnote der ursprünglichen Colognebase, eine würzig-krautig-holzige Note wird hervorgehoben.

**Patentansprüche**

1. Verbindungen der Formel

$$R^4-\overset{R^3}{\overset{|}{C}}(H)-\overset{R^2}{\overset{|}{CH}}-\overset{R^1}{\overset{|}{CH}}-\overset{OR}{\overset{|}{CH}}-\overset{CH_3}{\overset{|}{C}}=CH-CH_2-CH_3 \qquad (I)$$

worin R $C_{1-4}$-Alkyl, 1-$C_{1-2}$-Alkoxy-$C_{1-4}$-alkyl oder $C_{2-4}$-Alkenyl bedeutet, $R^1$, $R^2$ und $R^3$ Wasserstoff, Methyl oder Äthyl darstellen und $R^4$ Wasserstoff, $C_{1-5}$-Alkyl oder $C_{2-4}$-Alkenyl oder $C_{1-4}$-Alkyliden darstellt, wobei 2 oder 3 der Reste $R^1$, $R^2$, $R^3$ und $R^4$ Wasserstoff sind.

2. 5-Methoxy-4-methyl-3-decen.

3. 5-Methoxy-2,6-dimethyl-6-nonen.

4. 5-Äthoxy-2,6-dimethyl-6-nonen.

5. 5-(1'-Äthoxyäthoxy)-4-methyl-3-decen.

6. 5-(1'-Äthoxyäthoxy)-2,6-dimethyl-6-nonen.

7. Eine Verbindung ausgewählt aus 5-Methoxy-4,6-dimethyl-3-octen, 5-Methoxy-4,6-dimethyl-3-nonen, 5-Methoxy-4,7-dimethyl-3-nonen, 5-Methoxy-4-methyl-3-undecen, 5-Äthoxy-4-methyl-3-undecen, 5-Methoxy-4-methyl-3-tridecen, 5-Methoxy-2,6-dimethyl-1,6-nonadien, 5-Methoxy-4-methyl-3,8-(Z)-undecadien, 5-Äthoxy-4-methyl-3-decen, 5-Butoxy-4-methyl-3-decen, 5-Allyloxy-4-methyl-3-decen, 5-(1'-Methoxy-1'-methyläthoxy)-4-methyl-3-decen, 5-(1'-Methoxy-1'-methyläthoxy)-2,6-dimethyl-6-nonen, 5-(1'-Äthoxyäthoxy)-4-methyl-3-undecen.

8. Riech- und/oder Geschmackstoffkomposition, gekennzeichnet durch einen Gehalt an einer Verbindung der Formel

$$R^4-\overset{R^3}{\overset{|}{C}}(H)-\overset{R^2}{\overset{|}{CH}}-\overset{R^1}{\overset{|}{CH}}-\overset{OR}{\overset{|}{CH}}-\overset{CH_3}{\overset{|}{C}}=CH-CH_2-CH_3 \qquad (I)$$

worin R $C_{1-4}$-Alkyl, 1-$C_{1-2}$-Alkoxy-$C_{1-4}$-alkyl oder $C_{2-4}$-Alkenyl bedeutet, $R^1$, $R^2$ und $R^3$ Wasserstoff, Methyl oder Äthyl darstellen und $R^4$ Wasserstoff, $C_{1-5}$-Alkyl oder $C_{2-4}$-Alkenyl oder $C_{1-4}$-Alkyliden

darstellt, wobei 2 oder 3 der Reste $R^1$, $R^2$, $R^3$ und $R^4$ Wasserstoff sind.

9. Riech- und/oder Geschmackstoffkomposition, gekennzeichnet durch einen Gehalt an 5-Methoxy-4-methyl-3-decen.

10. Riech- und/oder Geschmackstoffkomposition, gekennzeichnet durch einen Gehalt an 5-Methoxy-2,6-dimethyl-6-nonen.

11. Riech- und/oder Geschmackstoffkomposition, gekennzeichnet durch einen Gehalt an 5-Äthoxy-2,6-dimethyl-6-nonen.

12. Riech- und/oder Geschmackstoffkomposition, gekennzeichnet durch einen Gehalt an 5-(1'-Äthoxyäthoxy)-4-methyl-3-decen.

13. Riech- und/oder Geschmackstoffkomposition, gekennzeichnet durch einen Gehalt an 5-(1'-Äthoxyäthoxy)-2,6-dimethyl-6-nonen.

14. Verfahren zur Herstellung der Verbindungen der Formel

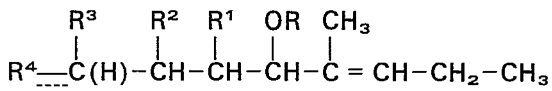

(I)

worin R $C_{1-4}$-Alkyl, 1-$C_{1-2}$-Alkoxy-$C_{1-4}$-alkyl oder $C_{2-4}$-Alkenyl bedeutet, $R^1$, $R^2$ und $R^3$ Wasserstoff, Methyl oder Äthyl darstellen und $R^4$ Wasserstoff, $C_{1-5}$-Alkyl oder $C_{2-4}$-Alkenyl oder $C_{1-4}$-Alkyliden darstellt, wobei 2 oder 3 der Reste $R^1$, $R^2$, $R^3$ und $R^4$ Wasserstoff sind, dadurch gekennzeichnet, dass man eine Verbindung der Formel

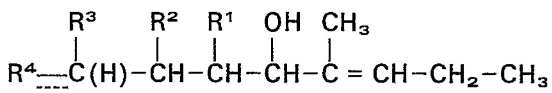

(II)

worin $R^1$, $R^2$, $R^3$ und $R^4$ obige Bedeutung besitzen, unter Einführung eines Restes R auf an sich bekannte Weise veräthert.

15. Verwendung von Verbindungen der Formel

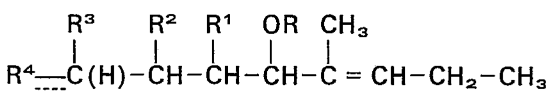

(I)

worin R $C_{1-4}$-Alkyl, 1-$C_{1-2}$-Alkoxy-$C_{1-4}$-alkyl oder $C_{2-4}$-Alkenyl bedeutet, $R^1$, $R^2$ und $R^3$ Wasserstoff, Methyl oder Äthyl darstellen und $R^4$ Wasserstoff, $C_{1-5}$-Alkyl oder $C_{2-4}$-Alkenyl oder $C_{1-4}$-Alkyliden darstellt, wobei 2 oder 3 der Reste $R^1$, $R^2$, $R^3$ und $R^4$ Wasserstoff sind, als Riech- und/oder Geschmackstoffe.

16. Verwendung von 5-Methoxy-4-methyl-3-decen als Riech und/oder Geschmackstoff.

17. Verwendung von 5-Methoxy-2,6-dimethyl-6-nonen, als Riech- und/oder Geschmackstoff.

18. Verwendung von 5-Äthoxy-2,6-dimethyl-6-nonen, als Riech- und/oder Geschmackstoff.

19. Verwendung von 5-(1'-Äthoxyäthoxy)-4-methyl-3-decen, als Riech- und/oder Geschmackstoff.

20. Verwendung von 5-(1'-Äthoxyäthoxy)-2,6-dimethyl-6-nonen, als Riech- und/oder Geschmackstoff.

## Claims

1. Compounds of the formula

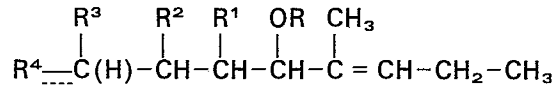

(I)

wherein R signifies $C_{1-4}$-alkyl, 1-$C_{1-2}$-alkoxy-$C_{1-4}$-alkyl or $C_{2-4}$-alkenyl, $R^1$, $R^2$ and $R^3$ represent hydrogen, methyl or ethyl and $R^4$ represents hydrogen, $C_{1-5}$-alkyl or $C_{2-4}$-alkenyl or $C_{1-4}$-alkylidene, whereby 2 or 3 of the residues $R^1$, $R^2$, $R^3$ and $R^4$ are hydrogen.

2. 5-Methoxy-4-methyl-3-decene.

3. 5-Methoxy-2.6-dimethyl-6-nonene.

4. 5-Ethoxy-2.6-dimethyl-6-nonene.

5. 5-(1'-Ethoxyethoxy)-4-methyl-3-decene.

6. 5-(1'-Ethoxyethoxy)-2.6-dimethyl-6-nonene.

7. A compound selected from 5-methoxy-4.6-dimethyl-3-octene, 5-methoxy-4.6-dimethyl-3-nonene, 5-methoxy-4.7-dimethyl-3-nonene, 5-methoxy-4-methyl-3-undecene, 5-ethoxy-4-methyl-3-undecene, 5-methoxy-4-methyl-3-tridecene, 5-methoxy-2.6-dimethyl-1.6-nonadiene, 5-methoxy-4-methyl-3.8-(Z)-undecadiene, 5-ethoxy-4-methyl-3-decene, 5-butoxy-4-methyl-3-decene, 5-allyloxy-4-methyl-3-decene, 5-(1'-methoxy-1'-methylethoxy)-4-methyl-3-decene, 5-(1'-methoxy-1'-methylethoxy)-2.6-dimethyl-6-nonene, 5-(1'-ethoxyethoxy)-4-methyl-3-undecene.

8. An odorant and/or flavouring substance composition, characterized by a content of a compound of the formula

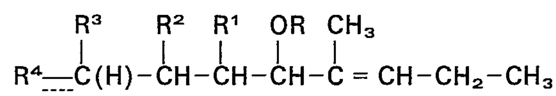

(I)

wherein R signifies $C_{1-4}$-alkyl, 1-$C_{1-2}$-alkoxy-$C_{1-4}$-alkyl or $C_{2-4}$-alkenyl, $R^1$, $R^2$ and $R^3$ represent hydrogen, methyl or ethyl and $R^4$ represents hydrogen, $C_{1-5}$-alkyl or $C_{2-4}$-alkenyl or $C_{1-4}$-alkylidene, whereby 2 or 3 of the residues $R^1$, $R^2$, $R^3$ and $R^4$ are hydrogen.

9. An odorant and/or flavouring substance composition, characterized by a content of 5-methoxy-4-methyl-3-decene.

10. An odorant and/or flavouring substance composition, characterized by a content of 5-methoxy-2.6-dimethyl-6-nonene.

11. An odorant and/or flavouring substance composition, characterized by a content of 5-ethoxy-2.6-dimethyl-6-nonene.

12. An odorant and/or flavouring substance composition, characterized by a content of 5-(1'-ethoxyethoxy)-4-methyl-3-decene.

13. An odorant and/or flavouring substance composition, characterized by a content of 5-(1'-ethoxyethoxy)-2.6-dimethyl-6-nonene.

14. A process for the manufacture of the compounds of the formula

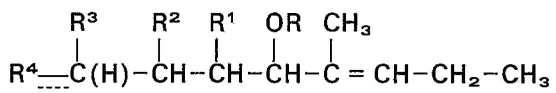

(I)

wherein R signifies $C_{1-4}$-alkyl, 1-$C_{1-2}$-alkoxy-$C_{1-4}$-alkyl or $C_{2-4}$-alkenyl, $R^1$, $R^2$ and $R^3$ represent hydrogen, methyl or ethyl and $R^4$ represents hydrogen, $C_{1-5}$-alkyl or $C_{2-4}$-alkenyl or $C_{1-4}$-alkylidene, whereby 2 or 3 of the residues $R^1$, $R^2$, $R^3$ and $R^4$ are hydrogen, characterized by etherifying a compound of the formula

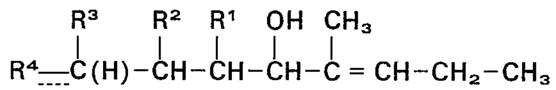

(II)

wherein $R^1$, $R^2$, $R^3$ and $R^4$ have the above significance, in a manner known *per se* with the introduction of a residue R.

15. The use of compounds of the formula

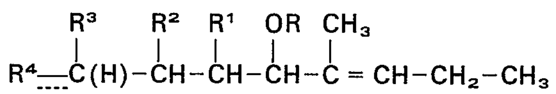

(I)

wherein R signifies $C_{1-4}$-alkyl, 1-$C_{1-2}$-alkoxy-$C_{1-4}$-alkyl or $C_{2-4}$-alkenyl, $R^1$, $R^2$ and $R^3$ represent hydrogen, methyl or ethyl and $R^4$ represents hydrogen, $C_{1-5}$-alkyl or $C_{2-4}$-alkenyl or $C_{1-4}$-alkylidene, whereby 2 or 3 of the residues $R^1$, $R^2$, $R^3$ and $R^4$ are hydrogen, as odorant and/or flavouring substances.

16. The use of 5-methoxy-4-methyl-3-decene as an odorant and/or flavouring substance.

17. The use of 5-methoxy-2.6-dimethyl-6-nonene as an odorant and/or flavouring substance.

18. The use of 5-ethoxy-2.6-dimethyl-6-nonene as an odorant and/or flavouring substance.

19. The use of 5-(1'-ethoxyethoxy)-4-methyl-3-decene as an odorant and/or flavouring substance.

20. The use of 5-(1'-ethoxyethoxy)-2.6-dimethyl-6-nonene as an odorant and/or flavouring substance.

## Revendications

1. Composés de formule

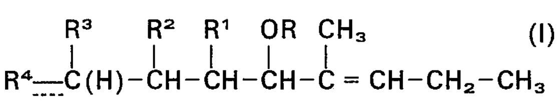

(I)

dans laquelle R représente un groupe alkyle en $C_{1-4}$, 1-(alcoxy en $C_{1-2}$)-alkyle en $C_{1-4}$ ou alcényle en $C_{2-4}$, $R^1$, $R^2$ et $R^3$ représentent l'hydrogène, des groupes méthyle ou éthyle et $R^4$ représente l'hydrogène, un groupe alkyle en $C_{1-5}$ ou alcényle en $C_{2-4}$ ou alkylidène en $C_{1-4}$, étant spécifié que deux ou trois des symboles $R^1$, $R^2$, $R^3$ et $R^4$ représentent l'hydrogène.

2. Le 5-méthoxy-4-méthyl-3-décène.

3. Le 5-méthoxy-2,6-diméthyl-6-nonène.

4. Le 5-éthoxy-2,6-diméthyl-6-nonène.

5. Le 5-(1'-éthoxyéthoxy)-4-méthyl-3-décène.

6. Le 5-(1'-éthoxyéthoxy)-2,6-diméthyl-6-nonène.

7. Un composé choisi parmi le 5-méthoxy-4,6-diméthyl-3-octène, le 5-méthoxy-4,6-diméthyl-3-nonène, le 5-méthoxy-4,7-diméthyl-3-nonène, le 5-méthoxy-4-méthyl-3-undécène, le 5-éthoxy-4-méthyl-3-undécène, le 5-méthoxy-4-méthyl-3-tridécène, le 5-méthoxy-2,6-diméthyl-1,6-nonadiène, le 5-méthoxy-4-méthyl-3,8-(Z)-undécadiène, le 5-éthoxy-4-méthyl-3-décène, le 5-butoxy-4-méthyl-3-décène, le 5-allyloxy-4-méthyl-3-décène, le 5-(1'-méthoxy-1'-méthyléthoxy)-4-méthyl-3-décène, le 5-(1'-méthoxy-1'-méthyléthoxy)-2,6-diméthyl-6-nonène, le 5-(1'-éthoxyéthoxy)-4-méthyl-3-undécène.

8. Composition odorante et/ou aromatisante, caractérisée en ce qu'elle contient un composé de formule

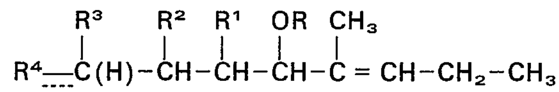

(I)

dans laquelle R représente un groupe alkyle en $C_{1-4}$, 1-(alcoxy en $C_{1-2}$)-alkyle en $C_{1-4}$ ou alcényle en $C_{2-4}$, $R^1$, $R^2$ et $R^3$ représentent l'hydrogène, des groupes méthyle ou éthyle et $R^4$ l'hydrogène, un groupe alkyle en $C_{1-5}$ ou alcényle en $C_{2-4}$ ou alkylidène en $C_{1-4}$, étant spécifié que deux ou trois des symboles $R^1$, $R^2$, $R^3$ et $R^4$ représentent l'hydrogène.

9. Composition odorante et/ou aromatisante, caractérisée en ce qu'elle contient du 5-méthoxy-4-méthyl-3-décène.

10. Composition odorante et/ou aromatisante, caractérisée en ce qu'elle contient du 5-méthoxy-2,6-diméthyl-6-nonène.

11. Composition odorante et/ou aromatisante, caractérisée en ce qu'elle contient du 5-éthoxy-2,6-diméthyl-6-nonène.

12. Composition odorante et/ou aromatisante, caractérisée en ce qu'elle contient du 5-(1'-éthoxyéthoxy)-4-méthyl-3-décène.

13. Composition odorante et/ou aromatisante, caractérisée en ce qu'elle contient du 5-(1'-éthoxyéthoxy)-2,6-diméthyl-6-nonène.

14. Procédé de préparation des composés de formule

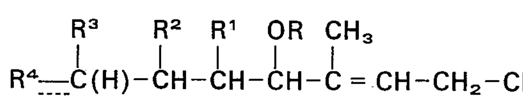

(I)

dans laquelle R représente un groupe alkyle en $C_{1-4}$, 1-(alcoxy en $C_{1-2}$)-alkyle en $C_{1-4}$ ou alcényle en $C_{2-4}$, $R^1$, $R^2$ et $R^3$ représentent l'hydrogène, des groupes méthyle ou éthyle et $R^4$ représente l'hydrogène, un groupe alkyle en $C_{1-5}$ ou alcényle en $C_{2-4}$ ou alkylidène en $C_{1-4}$, étant spécifié que deux ou trois des symboles $R^1$, $R^2$, $R^3$ et $R^4$ représentent l'hydrogène, caractérisé en ce que l'on éthérifie de manière connue en soi, avec introduction d'un reste R, un composé de formule

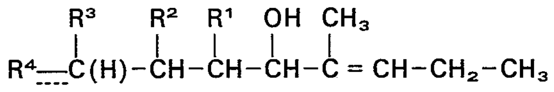

(II)

dans laquelle $R^1$, $R^2$, $R^3$ et $R^4$ ont les significations indiquées ci-dessus.

15. Utilisation des composés de formule

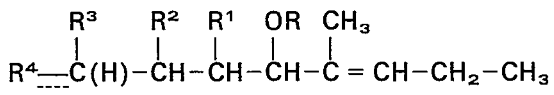

(I)

dans laquelle R représente un groupe alkyle en $C_{1-4}$, 1-(alcoxy en $C_{1-2}$)-alkyle en $C_{1-4}$ ou alcényle en $C_{2-4}$, $R^1$, $R^2$ et $R^3$ représentent l'hydrogène, des groupes méthyle ou éthyle et $R^4$ représente l'hydrogène, un groupe alkyle en $C_{1-5}$ ou alcényle en $C_{2-4}$ ou alkylidène en $C_{1-4}$, étant spécifié que deux ou trois des symboles $R^1$, $R^2$, $R^3$ et $R^4$ représentent l'hydrogène, en tant que substances odorantes et/ou aromatisantes.

16. Utilisation du 5-méthoxy-4-méthyl-3-décène en tant que substance odorante et/ou aromatisante.

17. Utilisation du 5-méthoxy-2,6-diméthyl-6-nonène en tant que substance odorante et/ou aromatisante.

18. Utilisation du 5-éthoxy-2,6-diméthyl-6-nonène en tant que substance odorante et/ou aromatisante.

19. Utilisation du 5-(1'-éthoxyéthoxy)-4-méthyl-3-décène en tant que substance odorante et/ou aromatisante.

20. Utilisation du 5-(1'-éthoxyéthoxy)-2,6-diméthyl-6-nonène en tant que substance odorante et/ou aromatisante.